# EUROPEAN PATENT SPECIFICATION

(11) **EP 0 634 656 B1**
(45) Date of publication and mention of the grant of the patent: **12.01.2000**
(21) Application number: 94111388.8
(22) Date of filing: 27.11.1987
(51) Int. Cl.: G01N 33/50, C12Q 1/00, C12Q 1/37

(54) **Processes for collecting body fluid from insects**
Verfahren zum Sammeln von Insekten-Körperflussigkeiten
Procédés pour rassembler des liquides corporels des insectes

(30) Priority: 03.12.1986 JP 28824486; 03.12.1986 JP 28824586
(43) Date of publication of application: 18.01.1995
(62) Divisional of application: 87117621.0
(73) Proprietor: WAKO PURE CHEMICAL INDUSTRIES, LTD., Higashi-ku Osaka (JP)
(72) Inventor: Ashida, Masaaki, Nishi-ku, Sapporo-shi (JP); Tsuchiya, Masakazu, Itami-shi (JP); Sakata, Yoshitsugu, Otsu-shi (JP); Matsuura, Shuji, Kawanishi-shi (JP)
(74) Representative: Baillie, Iain Cameron

(56) References cited:
- INSECT BIOCHEMISTRY, vol.15, no.6, 1985 pages 827 - 834 B.DULARAY ET AL. 'Haemolytic encapsulation and the prophenoloxidase-activation pathway in the locust Schistocerca Gregaria forsk'
- INSECT BIOCHEMISTRY, vol.11, no.1, 1981 pages 57 - 65 M.ASHIDA ET AL. 'A cane sugar factor suppressing activation of prophenoloxidase in haemolymph of the silkworm, Bombyx mori'
- H. Yoshida et M. Ashida, Insect. Biochem. vol. 16, No. 3, March 1986, p. 539 - 545

## Description

### BACKGROUND OF THE INVENTION

The body fluid of insects can be collected, for example, by the method disclosed by M. Ashida [Insect Biochem., 11, 57-65 (1981)]. In the mentioned method insects, for example, silkworm larvae, are anaesthetized on ice, and physiological saline containing either sugar including as an impurity cane sugar factor (polymeric substances included in sugar cane and comprising for example glucose, and amino acids), or cane sugar factor itself is injected into the haemocoel (body cavity) of the silkworm larvae. Then the larvae body is tied with a fine thread so as to prevent exudation of the injected saline. After being allowed to stand at room temperature for about 20 minutes, a cut is made into a leg and the body fluid is collected. The collected fluid is centrifuged to remove blood cells, then dialysed, and insect plasma is obtained.

Insect Biochemistry, Volume 16, No. 3, 1968 at page 539 "Microbial activation of two serine enzymes and prophenoloxidase in the plasma fraction of haemolymph of the silkworm, *Bombyx mori"* (Yoshida et al) discloses a method of obtaining insect body fluid including the step of contacting the haemolymph with a saline solution containing sugar and cane sugar factor, and subsequent dialysis of the haemolymph.

### SUMMARY OF THE INVENTION

In one embodiment, this invention provides a process for collecting a body fluid from an insect which comprises adding an insect body fluid to an isotonic solution for the insect to be used which contains a substance irreversibly inhibiting serine proteases, and removing an excess amount of the inhibiting substance.

In an alternative embodiment, this invention provides a process for collecting a body fluid from an insect which comprises;

injecting into the insect an isotonic solution for the insect to be used which contains a substance irreversibly inhibiting serine proteases, cutting into a part of the insect body, collecting the body fluid which comes out and removing an excess amount of the inhibiting substance.

### DESCRIPTION OF THE PREFERRED EMBODIMEHTS

There is no limitation to the insects which can be used in the process of this invention, but larger ones with known rearing methods therefor are preferable. Examples of such insects are Manduca serta, Gelleria melonella, Hyalphoma ceropia, and Bombyx mori (silkmoth), belonging to the Lepidoptera order; Sarcophaga peregrina, and musca, belonging to the Diptera order; Locusta, and migratoria Teleogryllus, belonging to the Orthoptera order; and Cerambyx belonging to the Coleoptera order.

Hemolymph which is the body fluid obtained from the body cavity is most easily obtained and thus usually used.

The body fluid of insects can be collected by the processes of the subject invention as described below. In one embodiment, to a solution isotonic to an insect body fluid to be collected and containing serine proteases (hereinafter referred to as "SP") inhibitor (hereinafter referred to as "SPI") which is a substance irreversibly inhibiting SP, an insect body fluid is added and then excess SPI is removed.

In an alternative embodiment there is described a process comprising the steps of injecting into the insect an isotonic solution for the insect to be used which contains a substance irreversibly inhibiting serine proteases, cutting into a part of the insect body, collecting the body fluid which comes out and removing an excess amount of the inhibiting substance.

As the SPI, there is no particular limitation thereto so long as it can irreversibly inhibit SP and it can be separated from the fraction of the insect body fluid which is obtained. Among the SPI's, preferable ones from the viewpoint of handling are those having relatively small molecular weights, e.g., 2000 or less, and being able to be removed by dialysis. Since cane sugar factor (CSF) cannot be removed by dialysis and has no suppressing effect in vitro, CSF is not an irreversibly acting SPI. Preferable examples of the SPI are (p-amizinophenyl)-methanesulfonyl fluoride (p-APMSF), phenylmethanesulfonyl fluoride (PMSF), diisopropylfluorophosphoric acid (DFP), p-nitrophenyl-p'-guanidinobenzoic acid (NPGB), and dihydroxychloromethylcoumalin.

It is sufficient to add to the above-mentioned isotonic solution the SPI in an amount at least necessary to inactivate SP which is to be inactivated during the collection of the body fluid. The preferable amount of SPI in the isotonic solution is 0.1 to 10 mM, more preferably 0.5 to 5 mM.

This method can be carried out, for example, as follows:

Body fluid coming out of an injured portion of the insect is directly dropped into a solution containing SPI, and isotonic to the insect body fluid to be collected. Alternatively, after injecting a solution containing the SPI and isotonic to the insect body fluid to be collected into an insect, the body fluid coming out of the injured portion of the insect is collected. Then, the collected fluid is centrifuged to remove blood cells. Further, excess SPI present in the resulting body fluid-containing solution is removed by one or more suitable methods generally used in the field of biochemistry for separation and purification depending on the nature of SPI used. Examples of such methods are a dialysis method, a gel filtration method, an ion exchange method, or high performance liquid chromatography. As a result, the desired solution containing insect plasma can be obtained. Further, the SPI removal operation can be conducted after subjecting the centrifuged solution to concentration and purification to some extent by a conventional method such as fractionation with ammonium sulfate.

This invention is illustrated by way of the following Examples, but not limited thereto.

### Reference Example 1

### Preparation of Silkworm Plasma

Silkworm plasma was prepared according to the Ashida method (Insect Biochem., 11, 57-65, 1981) as follows:

Silkworm larvae of the fifth instar were anaesthetized on ice for 10 min. An amount of half the body weight of physiological saline containing 20 mM of cane sugar or 6 µg/ml of cane sugar factor purified from sugar cane was injected into the haemocoel through a hypodermic needle inserted at the junction of the fifth and sixth abdominal segments. After the injection, the larvae body was tied with a fine thread at the posterior part of the fifth abdominal segment to prevent exudation of the injected saline. After having been allowed to stand at room temperature for 20 minutes, a leg was cut into to allow bleeding at the third abdominal segment. The collected hemolymph was centrifuged at 1500 g for 5 minutes at a low temperature (about 4°C) to remove haemocytes. The supernatant, in an amount of about 100 ml, the solution obtained was subjected to dialysis at a low temperature for 2 days against 3 liters of 0.01 M Tris-malate buffer (pH 6.5 containing 0.15 M KCL) to give the desired silkworm plasma.

### Example 1

Silkworm larvae (Bombyx mori) of the fifth instar were anaesthetized on ice for 10 minutes. A leg was cut into at the third abdominal segment and hemolymph coming out was dropped into 10 ml of a 0.9% NaCl solution containing 2 mM of p-APMSF and collected. About 15 ml of hemolymph was obtained from 25 silkworm larvae. From this, about 25 ml of a diluted solution of hemolymph was obtained. The diluted solution of hemolymph was centrifuged at 1500 g for 5 minutes to remove haemocytes. To 25 ml of the resulting supernatant, 37.5 ml of saturated ammonium sulfate solution was added to give a 60% saturated ammonium sulfate suspension, which was centrifuged at 10000 g for 20 minutes to give a precipitate. This precipitate was dissolved in 27 ml of 0.01 M Tris-malate buffer (pH 6.5, containing 0.15 M NaCl), to which was added 0.1 ml of a 200 mM p-APMSF solution and was dialyzed 4 times against 1 liter of 0.01 M Tris-malate buffer (pH 6.5, containing 0.15 M NaCl). The dialyzed solution was centrifuged at 10000 g for 20 minutes at low temperatures to give the desired silkworm plasma.

The same results were also obtained when NPGB was used in place of p-APMSF.

### Example 2

Silkworm larvae (Bombyx mori) of the fifth instar were anaesthetized on ice for 10 minutes. A 0.9% NaCl solution containing 2 mM of p-APMSF was injected into the haemocoel at the junction of the fifth and sixth abdominal segments. After the injection, the larvae body was tied with a fine thread at the posterior part of the fifth abdominal segment to prevent exudation of the injected saline. Then, a leg was cut into at the third abdominal segment to collect body fluid (hemolymph) which came out. The collected hemolymph was centrifuged at 1500 g for 5 minutes at low temperatures to remove haemocytes. To 10 ml of the obtained supernatant, 0.05 ml of a 200 mM p-APMSF solution was added and the solution obtained was centrifuged at 10000 g for 20 minutes at low temperatures. The supernatant was passed through a Sephadex G-25 column (2.5 cm in diameter and 25 cm long) and equilibrated with 0.01 M Tris-malate buffer (pH 6.5, containing 0.15 M NaCl). After collecting peaks of protein eluted into void volume of the column, the desired silkworm plasma was obtained.

## Claims

1. A process for collecting a body fluid from an insect which comprises;
adding an insect body fluid to a solution which is isotonic to the body fluid of said insect, said solution containing a substance irreversibly inhibiting serine proteases, and
removing an excess amount of said inhibiting substance.

2. A process for collecting a body fluid from an insect which comprises;
injecting into the insect an isotonic solution for the insect to be used which contains a substance irreversibly inhibiting serine proteases, cutting into a part of the insect body, collecting body fluid which comes out and removing an excess amount of said inhibiting substance.

## Patentansprüche

1. Verfahren zum Sammeln von Körperflüssigkeit von einem Insekt, welches Verfahren umfasst:
zusetzen einer Insekten-Körperflüssigkeit zu einer Lösung, die in Bezug auf die Körperflüssigkeit des Insekts isotonisch ist, wobei die Lösung eine Substanz enthält, die irreversibel Serinproteasen inhibiert, sowie
Entfernen einer überschüssigen Menge der inhibierenden Substanz.

2. Verfahren zum Sammeln von Körperflüssigkeit von einem Insekt, welches Verfahren umfasst:
Injizieren einer für das zu verwendende Insekt isotonischen Lösung in das Insekt, die eine Substanz enthält, die Serinproteasen irreversibel inhibiert; Einschneiden in einen Teil des Insektenkörpers; Sammeln der austretenden Körperflüssigkeit; und Entfernen einer überschüssigen Menge der inhibierenden Substanz.

## Revendications

1. Procédé pour recueillir un fluide corporel d'un insecte qui comprend :
l'ajout d'un fluide corporel d'insecte à une solution qui est isotonique par rapport au fluide corporel dudit insecte, ladite solution contenant une substance inhibant de manière irréversible les protéases de sérine, et
l'élimination d'un excès de ladite substance inhibitrice.

2. Procédé pour recueillir un fluide corporel d'un insecte qui comprend :
l'injection dans l'insecte d'une solution isotonique pour l'insecte à utiliser qui contient une substance inhibant de manière irréversible les protéases de sérine, l'incision sur une partie du corps de l'insecte, la récupération du fluide corporel qui s'écoule et l'élimination d'un excès de ladite substance inhibitrice.
